# EUROPEAN PATENT SPECIFICATION

(11) **EP 1 605 063 B1**
(45) Date of publication and mention of the grant of the patent: **01.08.2007**
(21) Application number: 05253472.4
(22) Date of filing: 06.06.2005
(51) Int. Cl.: C12Q 1/68, G01N 21/66, G01N 21/76

(54) **Hybridization detecting unit and DNA chip including the detecting unit**
Detektionseinheit für Hybridisierungen, sowie DNA-Chip umfassend die Detektionseinheit
unité de détection d'hybridisation et chip d'adn comprenant cette unité

(30) Priority: 07.06.2004 JP 2004168926
(43) Date of publication of application: 14.12.2005
(73) Proprietor: SONY CORPORATION, Tokyo 141 (JP)
(72) Inventor: Segwa, Yuji, Sony Corporation, Tokyo 141 (JP); Hirata, Tatsushiro Sony Corporation, Tokyo 141 (JP)
(74) Representative: Leppard, Andrew John

(56) References cited:
- WO-A-02/086162
- US-A1- 2001 021 534
- US-B1- 6 180 346
- US-B1- 6 673 533

## Description

The present invention relates to a hybridization detection technique, and a DNA chip including a detecting unit.

Recently, integrated substrates for bioassays referred to as so-called DNA chips or DNA micro-arrays (hereinafter referred to generically as "DNA chips" in the present application) on which predetermined DNAs are finely arranged by micro-array technology have come into use for analysis of gene mutation, analysis of SNPs (Single Nucleotide Polymorphism), analysis of frequency of gene expression, and the like, and have begun to be widely used in development of new drugs, clinical diagnosis, pharmacological genomics, studies of evolution, forensic medicine and other fields.

The DNA chip is characterized by enabling comprehensive analysis of hybridization because a wide variety and a large number of DNA oligo chains, cDNAs (complementary DNAs) or the like are integrated on a glass substrate or a silicon substrate.

Briefly describing an example of a method of analysis using the DNA chip, PCR amplification is performed by reverse transcription PCR reaction or the like of mRNA extracted from a cell, tissue or the like to DNA probes retained on a glass substrate or a silicon substrate while incorporating fluorescent probes dNTP, and after hybridization is performed on the substrate, fluorescence is measured by a predetermined detector.

Next, a technology relating to action of an electric field on a substance present in a charged state in a liquid phase is known. Specifically, a nucleic acid molecule is known to be elongated or moved under the action of an electric field in a liquid phase. According to its principles, phosphate ions (negative charge), which form a frame of the nucleic acid molecule, and hydrogen atoms (positive charge) resulting from ionization of water around the phosphate ions are considered to together form an ion cloud. A polarization vector (dipole) generated by the negative charge and the positive charge points in one direction as a whole as a result of application of a high-frequency high voltage, and consequently the nucleic acid molecule is elongated. In addition, when a non-uniform electric field in which lines of electric force concentrate at one part is applied, the nucleic acid molecule moves to the part where the lines of electric force concentrate (see Non-Patent document 1).

When a DNA solution is placed in a gap of a few ten to a few hundred µm between micro-electrodes and a high-frequency electric field of about 1 MV/m and about 1 MHz is applied to the DNA solution, dielectric polarization occurs in DNA present in a form of a random coil. As a result, the DNA molecule is elongated into a form of a straight line in parallel with the electric field. It is known that due to this electrodynamic effect referred to as "dielectrophoresis," the polarized DNA spontaneously draws to an electrode edge, and is then fixed on the electrode edge with one end of the DNA in contact with the electrode edge (see Non-Patent document 2).

A device and a method are proposed in which electrodes are provided in a reaction area, and an electric field is formed by the electrodes to apply an electrodynamic effect on nucleic acid molecules. For example, Patent document 1 discloses a technique for spreading a nucleic acid complex (double strand) with one part fixed to a substrate into a planar shape by using electrostatic attraction force or the like occurring between electric field forming electrodes and dipoles generated within nucleic acid by an electric field.

Patent document 2 discloses a technique for separating complementary strand sample DNA and noncomplementary strand sample DNA from each other by fixing a DNA probe to one of opposed electrodes and applying a direct-current voltage between the electrodes, and also using electrophoresis by a gel.

Patent document 3 proposes a DNA chip having a structure in which an oligonucleotide is fixed to an electrode pin.

US 2001/0021534 A1 discloses an apparatus for multi-array, multi-specific electrochemiluminescence (ECL) testing. The apparatus has reaction zones in which ECL-tagged analytes of interest may bind with binding agents. Following the binding reaction, an electrical potential is applied to the reaction zones to cause ECL where the analyte has bound to the binding agent. US 6,673,533 describes a similar apparatus.

### [Non-Patent document 1]

Seiichi Suzuki, Takeshi Yamanashi, Shin-ichi Tazawa, Osamu Kurosawa, and Masao Washizu: "Quantitative analysis on electrostatic orientation of DNA in stationary AC electric field using fluorescence anisotropy," IEEE Transaction on Industrial Applications, Vol. 34, No. 1, pp. 75 to 83 (1998)

### [Non-Patent document 2]

Masao Washizu, "DNA handling while viewing," Visualization Information, Vol. 20, No. 76 (January 2000)

### [Patent document 1]

Japanese Patent Laid-open No. Hei 7-224086

### [Patent document 2]

Japanese Patent Laid-open No. 2002-168864

### [Patent document 3]

Japanese Patent Laid-open No. 2001-242135

In short, various DNA chip techniques now proposed can be said to be techniques that set a reaction area providing a field for interaction between substances, that is, a field for hybridization on a substrate in advance, and fix nucleic acid for detection such as probe DNA or the like in the reaction area, to analyze hybridization as interaction between the nucleic acid for detection and complementary target nucleic acid.

However, the conventional DNA chip techniques present fundamental technical problems in that an operation of fixing nucleic acid for detection such as DNA probes or the like and hybridization take a long time. In addition, the conventional DNA chip techniques have technical problems in that on a detection surface, there is a deviation in integrating density (fixing density) of the nucleic acid for detection such as DNA probes or the like and a detected amount of hybridization is non-uniform.

It is accordingly desirable to provide a hybridization detecting unit and a DNA chip including the detecting unit that make it possible to solve the above technical problems.

According to an aspect of the present invention, there is provided a hybridization detecting unit (hereinafter abbreviated to a "detecting unit") including, a reaction area for providing a field for hybridization between nucleic acid for detection and target nucleic acid, and opposed electrodes disposed so that an electric field can be applied to a medium stored or retained in the reaction area, wherein an electrode surface of at least one of the opposed electrodes has depression parts and a power supply configured to apply an electric field to a medium in the reaction area so that nucleic acid for detection or target nucleic acid is moved into the depression parts so as to increase the concentration of the nucleic acid, and a DNA chip including at least the detecting unit.

The depression parts in the detecting unit can be used as a part for fixing the nucleic acid for detection or an area for making the hybridization progress. Further, by arranging the depression parts regularly at intervals on the order of nanometers, for example, it is possible to fix the nucleic acid for detection equally on the electrode surface functioning as a detection surface, and make a detected amount of hybridization on the electrode surface uniform.

In addition, embodiments of the present invention can prevent electrochemical reactions of an ionic solution that can be trapped in the reaction area, by coating the opposed electrodes with an insulating layer (for example an oxide film layer). When the insulating layer is provided, the depression parts may be formed in the insulating layer.

The opposed electrodes may be disposed on an upper side and a lower side with the reaction area interposed between the opposed electrodes, or disposed on a first side and a second side of the reaction area such that the reaction area is interposed between the opposed electrodes. The opposed electrodes may be disposed both on an upper side and a lower side and on a first side and a second side in some cases. Further, a plurality of opposed electrodes may be provided as required regardless of positions where the opposed electrodes are arranged. In any case, depression parts are formed on at least one of a pair of opposed electrodes as described above. Incidentally, depression parts may be formed on both electrodes forming the opposed electrodes according to the purpose.

When the opposed electrodes are disposed on an upper side and a lower side with the reaction area interposed between the opposed electrodes, optical pickup from the lower side of the reaction area can be performed by making at least the electrode on the lower side an electrode that transmits excitation light of a predetermined wavelength. That is, it is possible to integrate a device group related to the optical pickup in a region below the detecting unit, and integrate a device group for for example dropping a medium in a region above the reaction area. Thereby the device groups can be made compact.

The opposed electrodes function as means for forming an electric field in the medium. A direct-current electric field, an alternating-current electric field, a low-frequency electric field, or a high-frequency electric field may be able to be selected as the electric field according to the purpose. When a high-frequency alternating-current electric field in particular is applied, an electrodynamic effect of dielectrophoresis is obtained in a condition without occurrence of air bubbles due to electrolysis, whereby nucleic acid molecules present within the reaction area can be elongated (extended) and moved.

At the deepest portion of a depression part and a region around the deepest portion, in particular, an electric field concentrates and thus a non-uniform electric field is formed. It is thereby possible to move nucleic acid for detection to the region while elongating the nucleic acid for detection by dielectrophoresis, and thus complete a fixing operation in a short time.

In addition, by moving target nucleic acid to the deepest portion of the depression part and the region around the deepest portion by dielectrophoresis and thus increasing concentration of the target nucleic acid, and further elongating (extending) the target nucleic acid to adjust a higher order structure thereof and thus reduce steric hindrance at the time of hybridization, it is possible to increase hybridization efficiency. That is, hybridization can be made to progress quickly.

Main technical terms used in the present invention will be defined in the following.

"Nucleic acid for detection" may be a nucleic acid molecule present in a fixed state or a free state in a medium stored or retained in a reaction area and functioning as a probe (sensor) for detecting a nucleic acid molecule having a complementary base sequence specifically interacting with the nucleic acid molecule. A typical example is oligonucleotide or polynucleotide of DNA probes or the like.

"Target nucleic acid" may be a nucleic acid molecule having a base sequence complementary to the nucleic acid for detection.

"Nucleic acid" may refer to a polymer (nucleotide chain) of phosphoric ester of nucleosides resulting from glycosidic linkage between a purine or pyrimidine base and sugar, and widely includes oligonucleotides and polynucleotides including probe DNAs, DNAs (entire length or a fragment thereof) obtained by polymerizing purine nucleotides and pyrimidine nucleotides, cDNAs (c probe DNAs) obtained by reverse transcription, RNAs, polyamide nucleotide derivatives (PNA) and the like.

"Hybridization" may refer to reaction for forming complementary chains (a double strand) between complementary base sequence structures. "Mis-hybridization" refers to the complementary chain forming reaction that is not normal.

A "reaction area" may be an area that can provide a reaction field for interaction such as hybridization or the like, and includes a reaction field having a well shape that can store a liquid phase or gel, for example. The interaction occurring in the reaction area is not narrowly limited as long as an object and an effect of embodiments of the present invention are achieved. For example, it is possible to effect not only interaction between single stranded nucleic acids, that is, hybridization, but also interaction between peptide (or protein) and desired double stranded nucleic acid formed from nucleic acid for detection, enzyme response reaction, and other inter-molecular interactions. When the double stranded nucleic acid is used, for example, it is possible to analyze for example a bond between a receptor molecule such as a hormone receptor or the like as a transcription factor and a response element DNA portion.

"Opposed electrodes" may refer to at least one pair of electrodes disposed such that surfaces of the electrodes are opposed to each other. Incidentally, an "opposing axis" refers to an axis formed by a straight line connecting centers of surfaces of two opposed electrodes to each other.

A "depression part" may be a depression portion formed by microfabrication in an electrode or a surface of an insulating layer for coating the electrode, or a valley between projection portions formed on the surface, and refers to a part where an electric field concentrates. "Dielectrophoresis" may be a phenomenon in which a molecule is driven to a stronger electric field in a non-uniform electric field. Even when an alternating-current voltage is applied, the same driving effects as in a case of a direct-current voltage can be obtained because polarization polarity is reversed with reversal of polarity of the voltage applied (see Teru Hayashi (editor), "Micromachines and Materials Engineering (published by CMC)," pp. 37 to 46, Chapter 5 "Manipulation of Cells and DNA").

A "DNA chip" may refer to a substrate for hybridization detection where nucleic acid for detection such as DNA probes or the like is finely arranged in a free state or a fixed state, and includes a concept of a DNA micro-array.

According to embodiments of the present invention, it is possible to integrate (fix) gene information efficiently, and make hybridization progress efficiently in a short time.

Embodiments provide a hybridization detecting unit using an electrodynamic effect in a reaction area as a field for hybridization.

Further particular and preferred aspects of the present invention are set out in the accompanying independent and dependent claims. Features of the dependent claims may be combined with features of the independent claims as appropriate, and in combinations other than those explicitly set out in the claims.

The present invention will be described further, by way of example only, with reference to preferred embodiments thereof as illustrated in the accompanying drawings, in which:
FIG. 1 is a sectional view of a structure of principal parts of a first embodiment (1a) of a hybridization detecting unit;
FIG. 2 is a diagram schematically showing a structure of medium supplying means and optical pickup means for the detecting unit (1a);
FIG. 3 is an enlarged view of an example of the form and structure of a surface of an electrode (E₁ or E₂) in the detecting unit (1a);
FIG. 4 is an enlarged view of an example of a depression part formed on the surface of the electrode (E₁ or E₂);
FIG. 5 is an enlarged view of an example of another form and structure of an electrode surface part in the detecting unit (1a);
FIG. 6 is a diagram showing a modification of the electrode surface part;
FIG. 7 is a diagram showing another modification of the electrode surface part;
FIG. 8 is a diagram showing another modification of the electrode surface part;
FIG. 9 is a diagram showing another modification of the electrode surface part;
FIG. 10 is a diagram (a sectional view as viewed along a line I-I in FIG. 4) schematically showing a state of an electric field formed within a depression part (3);
FIG. 11 is a diagram schematically showing a state of an electric field formed within a depression part (31);
FIG. 12 is a diagram schematically showing a state of an electric field formed within a depression part (32);
FIG. 13 is a sectional view of a structure of principal parts of a second embodiment (1b) of a detecting unit according to the present invention;
FIG. 14 is a sectional view of a structure of principal parts of a third embodiment (1c) of a detecting unit (a sectional view as viewed along a line II-II in the figure is added);
FIG. 15 is a diagram showing an electrode form of a modification of the detecting unit (1c);
FIG. 16 is a diagram showing an example of a preferred embodiment of a DNA chip (10);
FIG. 17 is a diagram (drawing-substituting graph) showing distribution of electric field intensity of depressed and projected surfaces; and
FIG. 18 is a diagram (drawing-substituting graph) showing distribution of gradients of electric field intensity of electrode surfaces having depressions and projections.

### DETAILED DESCRIPTION OF THE PREFERRED EMBODIMENTS

Preferred embodiments of the present invention will hereinafter be described with reference to the accompanying drawings. Incidentally, each of embodiments shown in the accompanying drawings represents one example of a typical embodiment of the present invention, and is not construed as narrowing the scope of the present invention.

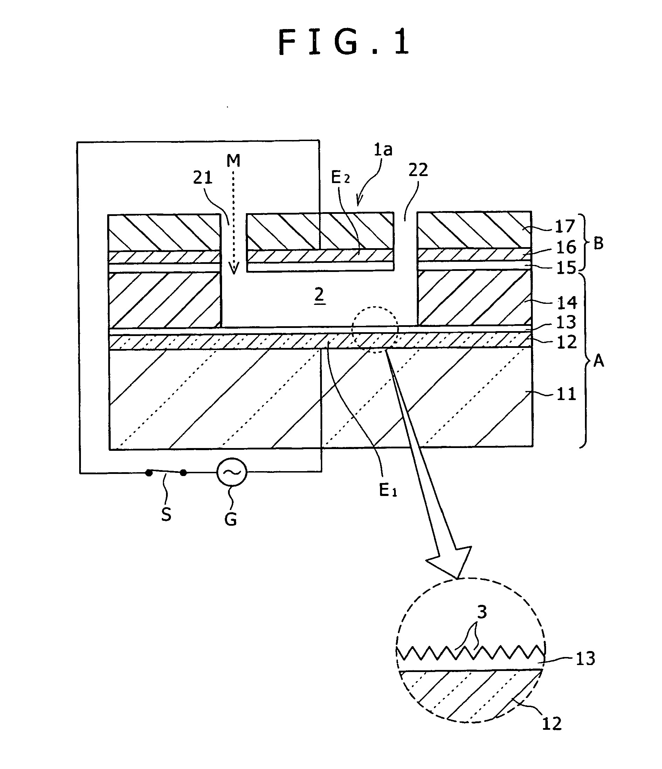

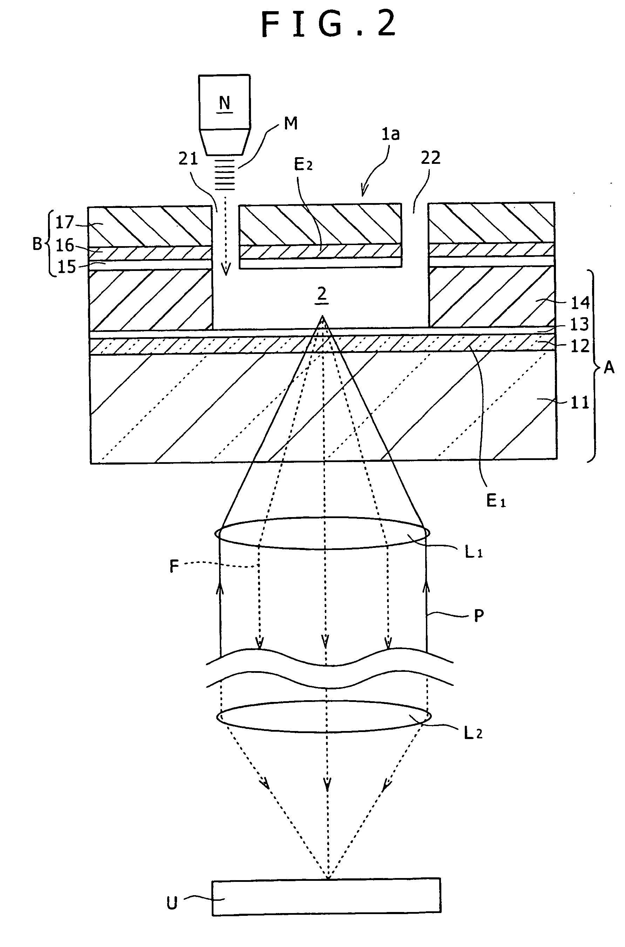

FIG. 1 is a sectional view of a structure of principal parts of a first embodiment of a hybridization detecting unit (hereinafter abbreviated to a "detecting unit"). FIG. 2 is a diagram schematically showing a structure of medium supplying means and optical pickup means for the detecting unit 1.

Reference 1a in FIG. 1 indicates a form of principal parts of the detecting unit. A DNA chip according to an embodiment of the present invention can be obtained by forming or arranging the detecting units 1a on a substrate.

The detecting unit 1a includes a lower side substrate A and an upper side substrate B placed on the lower side substrate A. The lower side substrate A includes a base material layer 11, a conductor layer 12 laminated on the base material layer 11, an insulating layer 13 laminated on the conductor layer 12, and a surface layer 14 laminated on the insulating layer 13 and having a well-shaped reaction area 2 formed by a predetermined surface treatment. The upper side substrate B includes a lower side insulating layer 15, a conductor layer 16 laminated on the insulating layer 15, and a top layer 17 laminated on the conductor layer 16.

The base material layer 11 of the lower side substrate A can be formed from a base material similar to that of an optical information recording medium such as a CD (Compact Disc), a DVD (Digital Versatile Disc), an MD (Mini Disc) or the like. The shape of the substrate according to embodiments of the present invention is not specifically limited. The substrate can be formed freely into a rectangular shape or a disc shape, for example.

The base material layer 11 is molded into a desired shape by quartz glass, silicon, or a synthetic resin such as polycarbonate, polystyrene or the like, preferably a synthetic resin capable of being injection-molded. Low running costs can be achieved by molding the substrate using an inexpensive synthetic resin as compared with conventionally used glass chips.

The conductor layer 12 is desirably formed of an optically transparent synthetic resin capable of transmitting excitation light P having a predetermined wavelength which light is used for hybridization detection (see FIG. 2). For example, the conductor layer 12 is desirably formed of aluminum or ITO (Indium Tin Oxide) as a conductor having optical transparency. The insulating layer 13 is formed of an inorganic substance such as SiO2, SiC, SiN, SiOC, SiOF, TiO₂ or the like. Incidentally, the insulating layer 13 (and the insulating layer 15) functions to prevent electrochemical reactions of an ionic medium that can be trapped in the reaction area 2, for example.

Such a structure of the lower side substrate A enables detection of hybridization within the reaction area 2 by irradiation with excitation light P from an underside (backside) of the substrate A (base material layer 11) as shown in FIG. 2.

Specifically, the excitation light P from the backside of the substrate enables detection of fluorescence F emitted from a target nucleic acid fluorescence-labeled in a hybridized state within the reaction area 2, or fluorescence F emitted from a fluorescent intercalator inserted into and bonded to a base pair forming complementary strands, by an optical system device group disposed on the underside (backside) of the substrate (see FIG. 2).

For example, the excitation light P as information reading light is emitted from a laser diode not shown in the figure, converted into collimated light by a collimator lens not shown in the figure, then advances and enters a condensing lens L₁ disposed under the substrate to be focused. FIG. 2 schematically shows a condensing lens for focusing the fluorescence F, which lens is denoted by reference L₂, and a detector for detecting the fluorescence F, which detector is denoted by reference U.

Assuming that design is made such that a device group necessary for an assay and detection is disposed around the detecting unit 1a or a DNA chip having the detecting unit la, a device group including a nozzle N for dropping or injecting a sample solution can be disposed en bloc in a region above the detecting unit 1a, and an optical device group for detection (readout) can be disposed en bloc in a region below the detecting unit 1a. As a result, a device group as a whole can be made compact.

The surface layer 14 forming the lower side substrate A can be formed by a photosensitive polyimide resin, for example. This photosensitive polyimide resin is subjected to surface treatment using a photoresist to form a minute reaction area 2.

While shape and size of the reaction area 2 are not specifically limited, length, width, and depth of the reaction area 2 are each a few *µ*m to a few hundred µm. This size value can be determined on the basis of a spot diameter of the excitation light and a minimum droppable amount of a medium M such as a sample solution (a solution including nucleic acid for detection or a solution including target nucleic acid) or the like.

Incidentally, the medium M is supplied by dropping a predetermined amount thereof via the nozzle N (for example an ink jet nozzle) from a hole 21 (or 22) formed in the upper side substrate B and communicating with the reaction area 2. Further, the medium M may be introduced into the reaction area 2 on principles using a capillary phenomenon or affinity for the medium M.

The conductor layer 12 (of the lower side substrate A) and the conductor layer 16 (of the upper side substrate B) in the detecting unit 1a are disposed on the lower side and the upper side such that the reaction area 2 is interposed between the conductor layer 12 and the conductor layer 16 (see FIG. 1 and FIG. 2). The conductor layer 12 and the conductor layer 16 function as opposed electrodes E₁ - E₂ for applying a desired electric field to the medium M stored or retained within the reaction area 2 via a power supply G by on/off operation of a switch S. Incidentally, it is desirable that the opposed electrodes E₁ - E₂ allow free selection of electric field intensity, selection of an alternating current or a direct current, or selection of a high-frequency electric field or a low-frequency electric field (the same is true for other opposed electrodes to be described below).

The opposed electrodes E₁ - E₂ function as means for elongating (extending) nucleic acid molecules (nucleic acid for detection or target nucleic acid) present in the medium M into a form of a straight chain, moving the nucleic acid molecules to a part on an electrode surface where lines of electric force concentrate, or moving or drawing the nucleic acid molecules while elongating (extending) the nucleic acid molecules, by application of a high-frequency alternating-current electric field, or more specifically by an electrodynamic effect of dielectrophoresis obtained by a sine wave with electric field conditions of about 1 × 10⁶ V/m and about 100 kHz to 100 MHz. Incidentally, an alternating-current electric field is more desirable than a direct-current electric field because the alternating-current electric field does not cause air bubbles due to electrolysis, and thus has small effects on a reaction system.

Hybridization generally progresses between single stranded nucleic acid molecules coiled in the form of a random coil or in an entangled state. Thus, when hybridization is affected by steric hindrance or the like, efficiency of complementary bonding is decreased, and mis-hybridization tends to occur.

However, an electric field applied via the opposed electrodes E₁ - E₂ can adjust the higher order structure of the nucleic acid molecules in an elongated (extended) state, or move the nucleic acid molecules to a predetermined region so as to increase probability of association (that is, concentration) of the nucleic acid molecules.

Hybridization in a state of an electric field being applied is not affected by steric hindrance, so that efficiency and accuracy of the hybridization can be dramatically increased. As a result, it is possible to realize high-speed hybridization and reduce mis-hybridization.

Incidentally, "steric hindrance" refers to a phenomenon in which desired reaction (hybridization in this case) does not occur easily because presence of a bulky substituent in the vicinity of a center of reaction or the like within a molecule or a position or three-dimensional structure (higher order structure) of the reacting molecule makes it difficult for another molecule to which the reaction is to occur to approach the reacting molecule.

In embodiments of the present invention, a form and structure of an electrode surface is devised so that the electrodynamic effect of the above-described dielectrophoresis is obtained more effectively so as to suit purposes of embodiments of the present invention. More specifically, an electrode surface of both or one of the electrodes E₁ and E₂ is formed such that depression parts 3 are regularly arranged at intervals on the order of nanometers. Incidentally, when the depression parts 3 are formed on both the electrodes E₁ and E₂, by fixing nucleic acid D for detection such as DNA probes to both the electrodes E₁ and E₂, it is possible to make hybridization progress on both the electrodes E₁ and E₂, and thereby increase an amount of fluorescence detected.

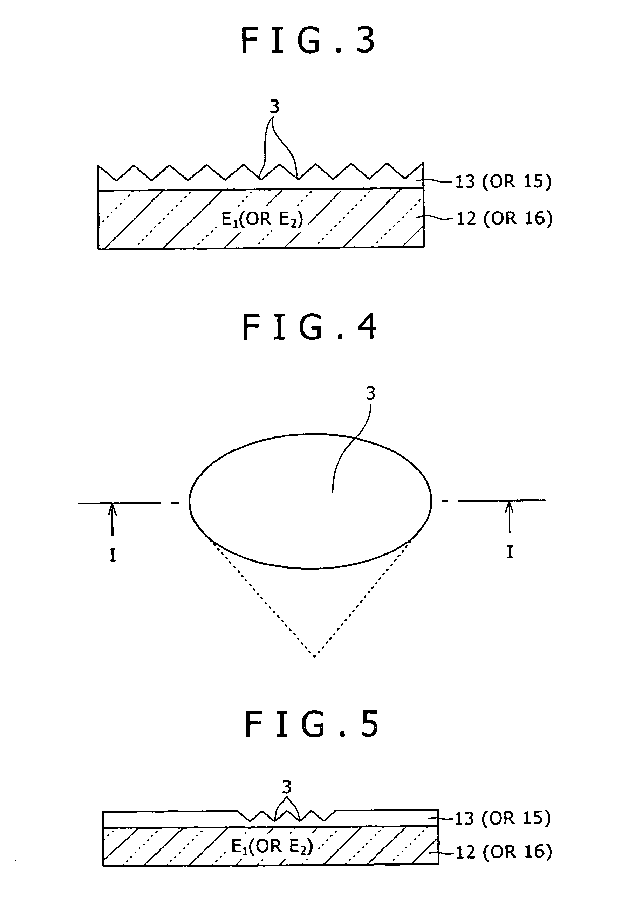

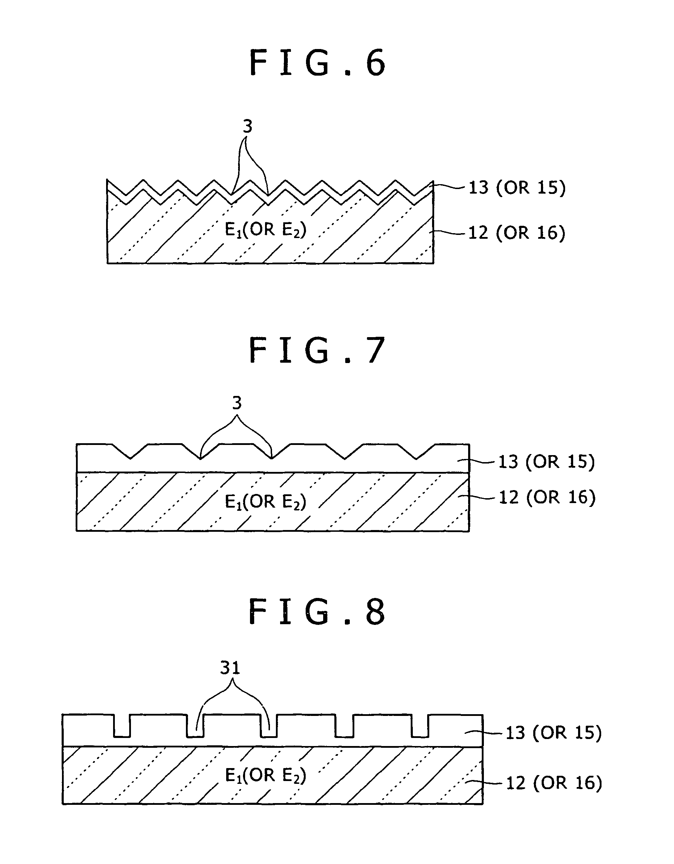

Preferred embodiments of the electrode surface will be described in the following with reference to FIGS. 3 to 9. FIG. 3 is an enlarged view of an example of the form and structure of the electrode surface in the detecting unit. FIG. 4 is an enlarged view of an example of a depression part formed on the electrode surface. FIG. 5 is an enlarged view of an example of another form and structure of an electrode surface part in the detecting unit. FIGS. 6 to 9 are a group of diagrams showing modifications of the form and structure.

First, as shown in FIG. 3, the part of the insulating layer 13 (or 15) corresponding to the surface of the electrode (E₁ or E₂) formed so as to face the reaction area 2 can be provided with continuous depression parts 3 having a cone shape (a section of a V-shape) as shown in FIG. 4, for example, over the entire surface of the electrode, or the depression parts 3 can be provided continuously in only one part of the insulating layer 13 (or 15) as shown in FIG. 5, for example.

Alternatively, a structure as in an embodiment shown in FIG. 6 can be employed in which the depression parts 3 are formed in advance in a layer functioning as an electrode, that is, the conductor layer 12 (or 16) itself, and the conductor layer 12 (or 16) is coated with the insulating layer 13 (or 15) such that forms of depression and projection formed by the depression parts 3 appear on the surface. This is true for the following embodiments shown in FIGS. 7 to 9.

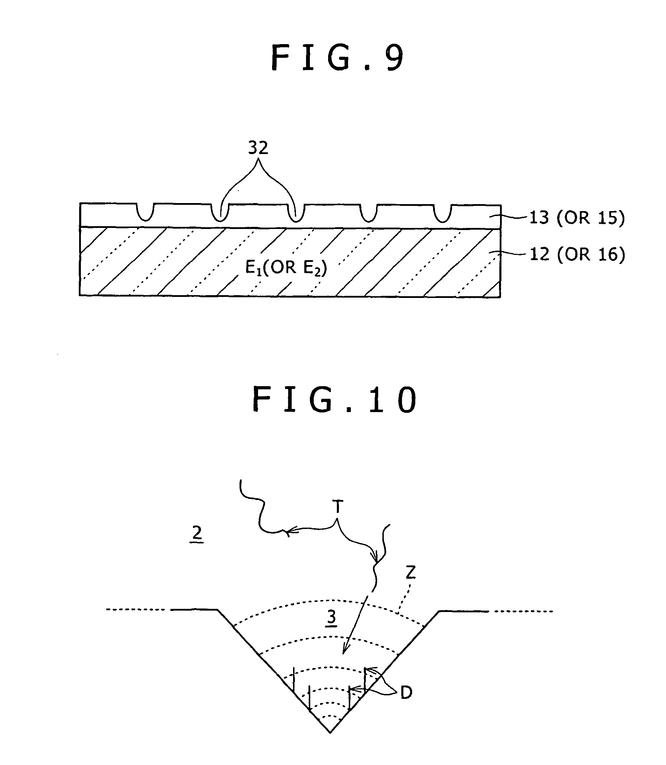

Further, it is possible to employ, as appropriate, for example a structure in which depression parts 3 having a section of a V-shape are formed at predetermined intervals, as shown in FIG. 7, a structure in which depression parts 31 having a section of a rectangular shape are formed at predetermined intervals, as shown in FIG. 8, or a structure in which depression parts 32 having a section of a U-shape are formed at predetermined intervals, as shown in FIG. 9.

Incidentally, a method of rough surface treatment for forming the depression parts 3 or the like on the electrode surface can be carried out by using widely known sputtering deposition techniques, epitaxy deposition techniques, and etching techniques, for example. However, the method itself is not particularly limited.

As is understood by reference to FIGS. 10 to 12, an electric field particularly concentrates and thus an electric field gradient is formed (that is, a non-uniform electric field is formed) in a part of a small space at a bottom, a bend or the like of the depression parts 3, 31, or 32 as described above.

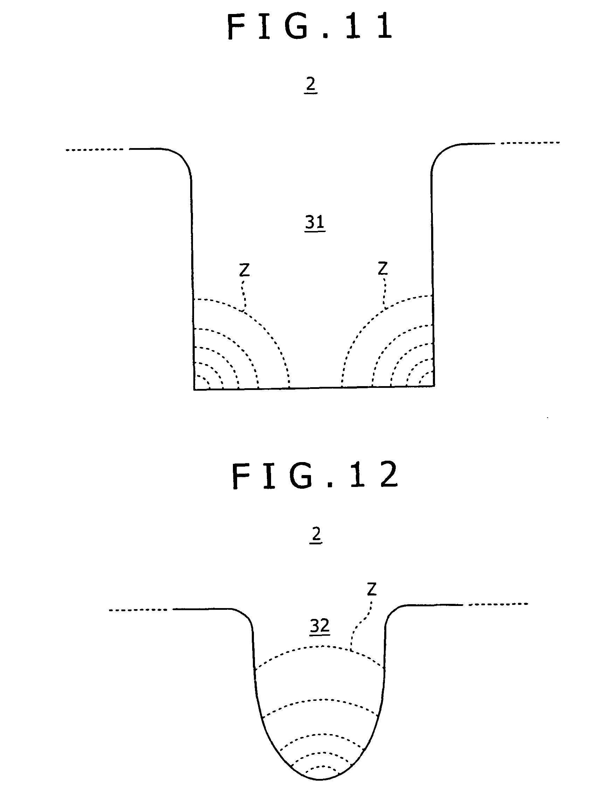

FIG. 10 is a diagram (a sectional view as viewed along a line I-I in FIG. 4) schematically showing a state of an electric field formed within a depression part 3. FIG. 11 is a diagram schematically showing a state of an electric field formed within a depression part 31. FIG. 12 is a diagram schematically showing a state of an electric field formed within a depression part 32. Reference Z shown in FIGS. 10 to 12 denotes a schematically represented distribution of electric field intensity.

A driving force caused by the electric field gradient acts on a dipole occurring in a nucleic acid molecule, whereby the nucleic acid molecule is moved into the depression part 3, 31, or 32 (into a part thereof where the electric field concentrates). When the nucleic acid molecule is nucleic acid D for detection, the nucleic acid molecule is drawn to the surface of the depression part 3 or the like, and is then fixed by a predetermined chemical bond in an elongated and aligned state.

FIG. 10 shows a state in which target nucleic acid T in a free state within the reaction area 2 is being moved by effects of dielectrophoresis toward the nucleic acid D for detection fixed at the bottom of the depression part 3 and extended by the electric field.

By thus disposing the depression parts 3 (or 31 or 32) regularly on the surface of the electrode (E₁ or E₂), it is possible to eliminate deviation in integrating density (fixing density) of the nucleic acid for detection such as DNA probes or the like. Further, since hybridization is made to progress in the depression parts 3 (or 31 or 32) disposed regularly on the surface of the electrode (E₁ or E₂), an amount of hybridization detected on the surface of the electrode (E₁ or E₂) can be made uniform.

Incidentally, hybridization can be detected by measuring intensity of fluorescence from a fluorochrome with which the target nucleic acid is labeled or a fluorescent intercalator having a characteristic of emitting fluorescence which intercalator is inserted into and bonded to a base pair part of double stranded nucleic acid. The fluorescent intercalator may be added to the reaction area 2 simultaneously with the target nucleic acid, or may be added after the progress of the hybridization. The use of the fluorescent intercalator has an advantage of being able to omit an operation of removing excess target nucleic acid from the reaction area 2 by washing.

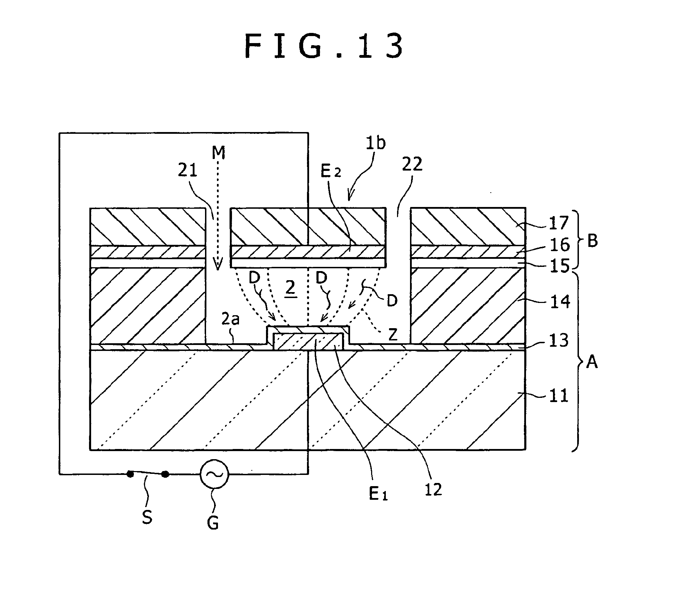

FIG. 13 is a sectional view of a structure of principal parts of a second embodiment of a detecting unit.

A detecting unit 1b according to the second embodiment is characterized in that one of opposed electrodes (E₁ in this case) is formed so as to have a smaller surface area than the electrode (E₂ in this case) on the other side.

This structure makes an electric field (lines of electric force) more concentrated and thus a non-uniform electric field formed at the electrode E₁ having the smaller surface area (see reference Z in FIG. 13). Therefore nucleic acid molecules are moved toward the electrode E₁ by dielectrophoresis. FIG. 13 schematically shows, as a typical example, a state in which nucleic acid D for detection in a free state is being moved toward the electrode E₁.

Thus, nucleic acid molecules spread widely in a free state within the reaction area 2 can be collected in a region around the electrode E₁, whereby concentration of the nucleic acid molecules can be maintained at a high level. Further, the nucleic acid molecules can be subsequently moved to a local part where an electric field concentrates more (a part where there is an electric field intensity gradient) within each depression part 3 (31 or 32) on the surface of the electrode E₁.

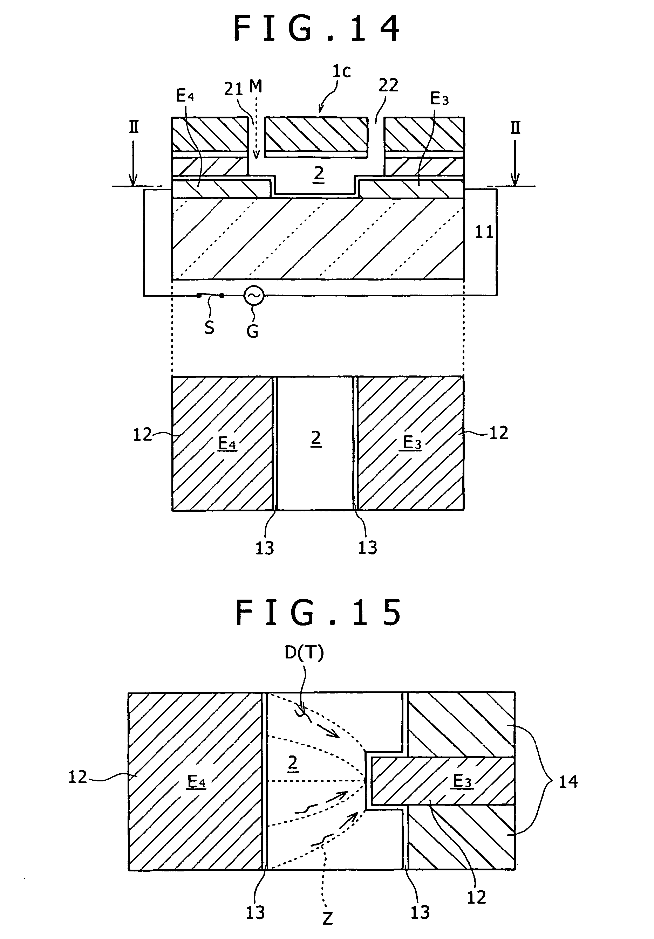

FIG. 14 is a sectional view of a structure of principal parts of a third embodiment of a detecting unit. In FIG. 14, a sectional view as viewed along a line II-II in the figure is added (see a lower diagram in FIG. 14).

A detecting unit 1c according to the third embodiment is characterized in that opposed electrodes E₃ - E₄ are allocated on a left and a right of a reaction area 2. FIG. 15 shows a modification of the detecting unit 1c which modification is characterized in that an electrode (E₃ in this case) used as an electrode to which to draw nucleic acid molecules is formed so as to have a smaller surface area than another electrode E₄.

As in the above-described detecting unit 1b, in this modification shown in FIG. 15, an electric field is more concentrated and thus a non-uniform electric field is formed at the electrode E₃ having the smaller surface area (see lines of electric force Z in FIG. 15). Therefore nucleic acid molecules (nucleic acid D for detection or target nucleic acid T) spread in a free state in a reaction area 2 are moved toward the electrode E₃ by dielectrophoresis. That is, it is possible to increase concentration of nucleic acid molecules in a region around the electrode E₃, and also draw the nucleic acid molecules into depression parts 3 (31 or 32).

Incidentally, though not shown specifically in the figures, the opposed electrodes E₁ - E₂ disposed on the upper and the lower side and the opposed electrodes E₃ - E₄ disposed on the left and the right side may be both disposed. In this case, the opposed electrodes E₁ - E₂ can be used as means for applying a high-frequency electric field to adjust the higher order structure of nucleic acid D for detection or improve hybridization efficiency, and the opposed electrodes E₃ - E₄ can be used as means for applying an electric field to forcefully remove excess substances hindering hybridization detection (for example excess nucleic acid D for detection, excess target nucleic acid T, excess intercalators and the like) and nucleic acid molecules resulting from mis-hybridization from a detection surface part to another region. A removal operation using such an electric field is a technique that can replace a washing removal operation using a water solution which operation is now commonly performed.

The surfaces of the electrodes E₁, E₂, E₃, and E₄ (forming opposed electrodes facing the reaction area 2) described above can be used as surfaces for fixing nucleic acid D for detection such as DNA probes or the like.

In order to fix desired nucleic acid D for detection such as DNA probes or the like, the electrode surface can be subjected in advance to surface treatment with a polylysine solution or a silane coupler solution containing an amino group, for example. When a substrate made of synthetic resin is subjected to surface treatment, the surface of the substrate is treated with a silane coupler solution containing an amino group after plasma treatment and DUV (Deep UV, far infrared radiation) irradiation treatment.

In addition, copper, silver, aluminum, or gold may be sputtered onto the electrode surface to form a film, and the surface layer may be coated with a substance having a functional group (active group) such as an amino group, a thiol group, a carboxyl group or the like, cysteamine, avidin (for example streptavidin), or the like.

A detection surface subjected to surface treatment with avidin is suitable for fixing a biotinylated DNA probe terminal. Alternatively, a detection surface treated with a thiol (SH) group is suitable for fixing nucleic acid D for detection such as probe DNA or the like having a terminal modified by a thiol group by a disulfide bond (-S-S- bond).

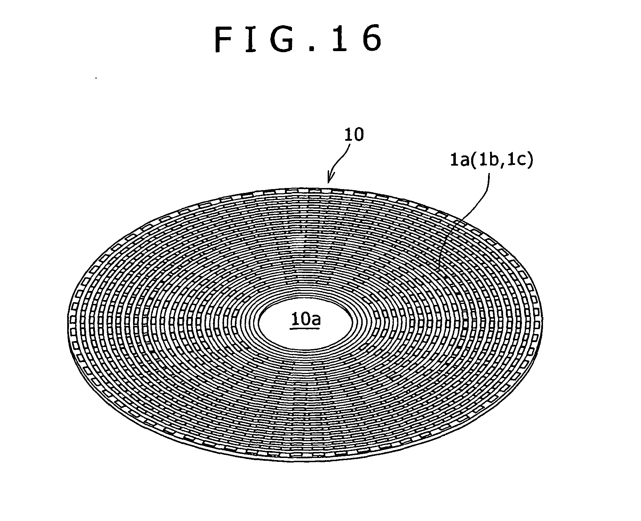

An example of a preferred embodiment of a DNA chip will next be described with reference to FIG. 16.

A DNA chip 10 shown in FIG. 16 has detecting units 1a (or 1b or 1c) described above arranged in a circumferential direction, in a form of a spiral, or in a radial manner, for example, on a substrate having a disc shape like a CD. An electric field can be applied to opposed electrodes provided in each detecting unit la via a current-carrying jig not shown in the figure inserted into a center hole 10a and feeding wiring disposed in the substrate.

The DNA chip 10 is rotated, a position is detected while a servo mechanism is operated, and a medium M can be supplied from above the substrate to a predetermined reaction area 2 by means of dropping or the like. In addition, hybridization detection can be performed by detecting fluorescence obtained by irradiating a predetermined reaction area 2 with excitation light P from below the substrate (backside of the substrate).

In order to verify forms of the electrode surface that allow nucleic acid molecules to be effectively drawn to the electrode surface by a dielectrophoresis phenomenon in a hybridization detection method, an experiment was conducted, and electric field intensity in the vicinity of the electrode surface and the like were calculated.

In a detecting unit as a calculation model, an ITO layer (lower electrode) as a conductor layer having a thickness of 250 nm was formed on a glass substrate, and further a SiO₂ layer (having a thickness of 150 nm and a dielectric constant ε = 5) as an insulating layer was formed on the ITO layer. In addition, depressions and projections having a height and depth of ±5 to 15 nm were formed at the surface of the lower electrode (an angle at which the depressions and projections were formed was 90°). A polyimide resin having a thickness of 5 µm was laminated on the SiO₂ (silicon dioxide) layer to form a well-shaped reaction area 2 (size: ϕ 100 µm). An upper electrode (material: SiO₂ (100 nm)/Al (100 nm)/Glass) is placed over the reaction area 2 so as to cover the reaction area 2.

Distributions of electric field intensity at the electrode surface when a voltage of 1 V was applied between the upper and lower opposed electrodes of the detecting unit as the calculation model were calculated. While an alternating-current electric field was applied between the electrodes in this experiment, the electric field was considered to be a static electric field in calculation for a limited purpose of grasping tendencies of distribution of electric field intensity. As for dielectric constants of materials required for electric field calculation, a dielectric constant of SiO₂ was ε = 5, and a dielectric constant of a buffer solution containing nucleic acid molecules (DNA), which solution was considered to be substantially the same as water, was ε = 78.

A calculation of distribution of electric field intensity in the vicinity of depressions and projections of the electrode surface verified that electric field intensity is gradually increased at the bottom of the depressions as compared with an upper region with an electric field gradient as shown in FIG. 10. On the other hand, it verified that electric field intensity is higher at the base of the projections than at the tip of the projections. That is, it was verified that an electric field gradient similar to that of a depression part is formed at a part of rising from a flat portion to a projecting portion.

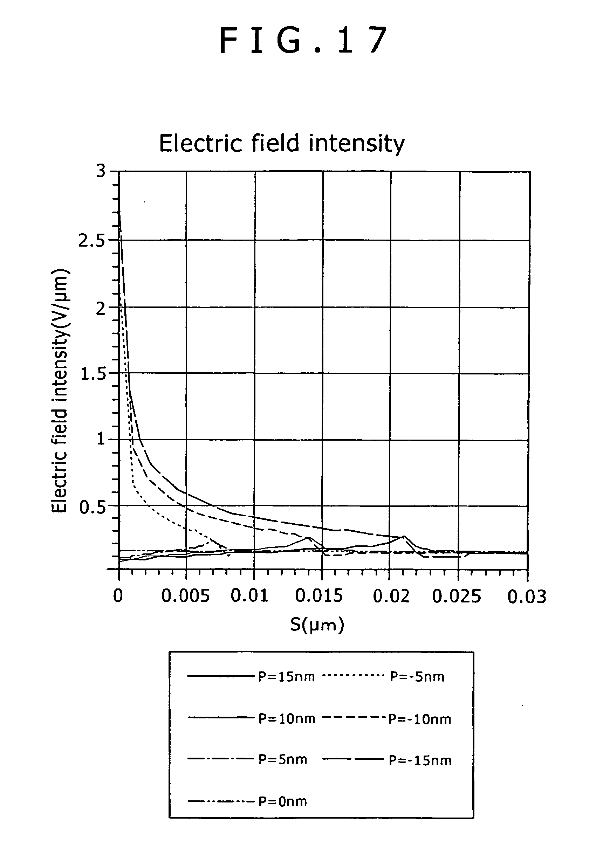

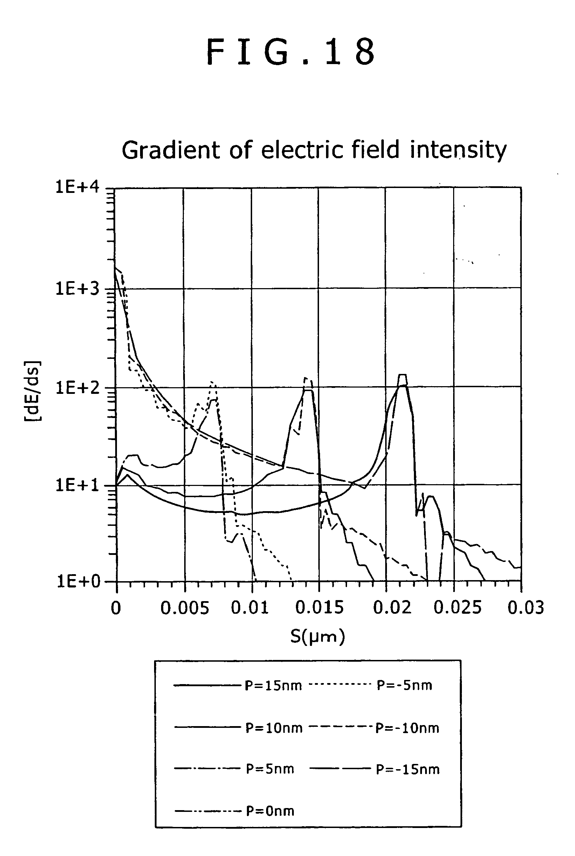

FIG. 17 and FIG. 18 are graphs of distribution of electric field intensity of the depression and projection surfaces and distribution of gradients of the electric field intensity. An axis of abscissas in FIG. 17 and FIG. 18 indicates distance (units: µm) in a horizontal direction from a maximum depth of a depression part or a projection part. An axis of ordinates in FIG. 17 indicates electric field intensity (units: V/µm). An axis of ordinates in FIG. 18 indicates gradients of electric field intensity. In FIG. 17 and FIG. 18, p = 5, 10, and 15 nm each denote a height position from a reference surface of the projection part, and p = -5, - 10, and -15 nm each denote a depth position from a reference surface of the depression part.

As shown in FIG. 17, it is clearly understood that the electric field intensity is obviously maximized at the bottom of the depression part, and that the value of the electric field intensity does not depends on depth. It is also understood from FIG. 18 that the gradient of the electric field intensity is also maximized at the bottom of the depression part. Thus, since the force of dielectrophoresis is increased in proportion to a square of the gradient of the electric field intensity, it became evident that the force of attraction of DNA is strongest at the bottom of the depression part.

It is also understood that forming the depression part is effective in attracting DNA by the force of dielectrophoresis than forming the projection part. Further, since the electric field intensity and the gradient of the electric field intensity do not depend on depth of the depression part, it suffices to form the depression part with a depth of a few to a few ten nm corresponding to length of a nucleic acid molecule whose hybridization is actually detected.

Thus, by employing a form in which depression parts are regularly arranged as shown in FIG. 3 and FIGS. 5 to 9 described above, for example, as an electrode surface, it is possible to integrate (fix) nucleic acid molecules at equal intervals on the electrode surface. Thereby steric hindrance and the like are alleviated, so that the time of hybridization on the electrode surface can be shortened, and a detected amount can be made uniform on the electrode surface.

When no electric field is applied, it takes about four hours or more to complete hybridization, whereas when the electric field is applied, a fluorescence signal equal to that at the completion of the hybridization was obtained in about five minutes. That is, high-speed hybridization can be achieved.

Thus, it is considered that a non-uniform electric field formed by a depression part on an electrode surface produces an effect of dielectrophoresis on target nucleic acid, that concentration of the target nucleic acid in the electrode surface region to which DNA probes as nucleic acid for detection are fixed is increased, and that hybridization reaction is consequently accelerated.

In hybridization detection, intercalators and fluorochrome with which target nucleic acid is labeled may be used, and also molecular beacons may be used.

The depth and the angle of depression parts on an electrode surface are not limited, and it is desirable to select suitable values on the basis of an amount, kind, and molecular length of nucleic acid molecules desired to be attracted (moved). Further, intensity and frequency of an electric field are not specifically limited, and it is desirable to select proper values on the basis of a kind, molecular length and the like of nucleic acid molecules. In addition, waveform is not limited to sine waves, and may be triangular waves or the like.

Embodiments of the present invention enables hybridization detection to be performed efficiently in a short time with a high detection accuracy. Embodiments of the present invention are particularly useful for techniques of detecting hybridization on a DNA chip or the like.

A hybridization detecting unit is provided including, a reaction area for providing a field for hybridization between nucleic acid for detection and target nucleic acid, and opposed electrodes disposed so that an electric field can be applied to a medium stored or retained in the reaction area, wherein an electrode surface of at least one of the opposed electrodes has depression parts.

## Claims

1. A hybridization detecting unit (1a, 1b, 1c) comprising:
a reaction area (2) for providing a field for hybridization between nucleic acid for detection (D) and target nucleic acid (T); and
opposed electrodes (E1;E2,E3;E4) disposed so that an electric field can be applied to a medium (M) stored or retained in the reaction area;
wherein an electrode surface of at least one of said opposed electrodes has depression parts (3,31,32); and
a power supply (G) configured to apply an electric field to a medium in the reaction area so that nucleic acid for detection or target nucleic acid is moved into the depression parts so as to increase the concentration of the nucleic acid.

2. The hybridization detecting unit as claimed in claim 1,
wherein said depression parts are parts for fixing said nucleic acid for detection.

3. The hybridization detecting unit as claimed in claim 1,
wherein said opposed electrodes are coated with an insulating layer (13,15).

4. The hybridization detecting unit as claimed in claim 3,
wherein said depression parts are formed in said insulating layer.

5. The hybridization detecting unit as claimed in claim 1,
wherein said depression parts are arranged regularly.

6. The hybridization detecting unit as claimed in claim 1,
wherein said opposed electrodes are disposed on an upper side and a lower side with said reaction area interposed between said opposed electrodes.

7. The hybridization detecting unit as claimed in claim 6,
wherein said depression parts are formed on at least one of said opposed electrodes.

8. The hybridization detecting unit as claimed in claim 6,
wherein at least said electrode on the lower side is an electrode that transmits excitation light (P) of a predetermined wavelength.

9. The hybridization detecting unit as claimed in claim 1,
wherein said opposed electrodes are disposed on a first side and a second side of the reaction area such that said reaction area is interposed between said opposed electrodes.

10. The hybridization detecting unit as claimed in claim 1,
wherein said opposed electrodes are formed so as to be able to apply a high-frequency alternating-current electric field to said medium.

11. A DNA chip (10) comprising at least the hybridization detecting unit of claim 1.

12. A method of detecting hybridization between nucleic acid for detection (D) and target nucleic acid (T) in a medium (M), comprising:
providing a hybridization detecting unit (1a, 1b, 1c) comprising:
a reaction area (2) for providing a field for hybridization between nucleic acid for detection and target nucleic acid; and
opposed electrodes (E1;E2,E3;E4) disposed so that an electric field can be applied to a medium stored or retained in the reaction area;
wherein an electrode surface of at least one of said opposed electrodes has depression parts (3,31,32);
introducing the medium into the reaction area; and
applying an electric field to the medium so that nucleic acid for detection or target nucleic acid is moved into the depression parts so as to increase the concentration of the nucleic acid.

## Patentansprüche

1. Hybridisierungs-Ermittlungseinheit (1a, 1b, 1c), die aufweist:
einen Reaktionsbereich (2) zum Liefern eines Felds zur Hybridisierung zwischen Kernsäure zur Ermittlung (D) und Zielkernsäure (T); und
gegenüberliegende Elektroden (E₁; E₂, E₃; E₄), die so angeordnet sind, dass ein elektrisches Feld an ein Medium (M) angelegt werden kann, welches im Reaktionsbereich aufbewahrt oder gehalten wird;
wobei eine Elektrodenfläche von zumindest einer der gegenüberliegenden Elektroden Vertiefungsteile (3, 31, 32) hat; und
eine Spannungsversorgung (G), die aufgebaut ist, ein elektrisches Feld an ein Medium im Reaktionsbereich anzulegen, so dass Kernsäure zur Ermittlung oder Zielkernsäure in die Vertiefungsteile bewegt wird, um somit die Konzentration der Kernsäure zu steigern.

2. Hybridisierungs-Ermittlungseinheit nach Anspruch 1,
wobei die Vertiefungsteile Teile sind, um die Kernsäure zur Ermittlung zu fixieren.

3. Hybridisierungs-Ermittlungseinheit nach Anspruch 1,
wobei die gegenüberliegenden Elektroden mit einer Isolationsschicht (13, 15) überzogen sind.

4. Hybridisierungs-Ermittlungseinheit nach Anspruch 3,
wobei die Vertiefungsteile in der Isolationsschicht gebildet sind.

5. Hybridisierungs-Ermittlungseinheit nach Anspruch 1,
wobei die Vertiefungsteile regelmäßig angeordnet sind.

6. Hybridisierungs-Ermittlungseinheit nach Anspruch 1,
wobei die gegenüberliegenden Elektroden auf einer oberen Seite und einer unteren Seite angeordnet sind, wobei der Reaktionsbereich zwischen den gegenüberliegenden Elektroden angeordnet ist.

7. Hybridisierungs-Ermittlungseinheit nach Anspruch 6,
wobei die Vertiefungsteile auf zumindest einer der gegenüberliegenden Elektroden gebildet sind.

8. Hybridisierungs-Ermittlungseinheit nach Anspruch 6,
wobei zumindest die Elektrode auf der unteren Seite eine Elektrode ist, welche Erregungslicht (P) einer vorher festgelegten Wellenlänge überträgt.

9. Hybridisierungs-Ermittlungseinheit nach Anspruch 1,
wobei die gegenüberliegenden Elektroden auf einer ersten Seite und einer zweiten Seite des Reaktionsbereichs angeordnet sind, so dass der Reaktionsbereich zwischen den gegenüberliegenden Elektroden angeordnet ist.

10. Hvbridisienmgs-Ermittlungseinheit nach Anspruch 1,
wobei die gegenüberliegenden Elektroden so gebildet sind, um in der Lage zu sein, ein hochfrequentes elektrisches Wechselstromfeld an das Medium anzulegen.

11. DNA-Chip (10), der zumindest die Hybridisierungs-Ermittlungseinheit von Anspruch 1 aufweist.

12. Verfahren zum Ermitteln von Hybridisierung zwischen Kernsäure zur Ermittlung (D) und Zielkernsäure (T) in einem Medium (M), welches aufweist:
Bereitstellen einer Hybridisierungs-Ermittlungseinheit (1a, 1b, 1c), welche aufweist:
einen Reaktionsbereich (2) zum Bereitstellen eines Felds zur Hybridisierung zwischen Kernsäure zur Ermittlung und Zielkernsäure; und
gegenüberliegende Elektroden (E₁; E₂, E3; E4), die so angeordnet sind, dass ein elektrisches Feld an ein Medium angelegt werden kann, welches im Reaktionsbereich aufbewahrt oder gehalten wird;
wobei eine Elektrodenfläche von zumindest einer der gegenüberliegenden Elektroden Vertiefungsteile (3, 31, 32) hat;
Einführen des Medium in den Reaktionsbereich; und
Anlegen eines elektrischen Felds an das Medium, so dass Kernsäure zur Ermittlung oder Zielkernsäure in die Vertiefungsteile bewegt wird, um somit die Konzentration der Kernsäure zu steigern.

## Revendications

1. Unité de détection d'hybridation (1a, 1b, 1c) comprenant :
une zone de réaction (2) destinée à fournir un champ pour l'hybridation entre l'acide nucléique pour la détection (D) et l'acide nucléique cible (T) ; et
des électrodes opposées (E1 ; E2, E3 ; E4) disposées de sorte qu'un champ électrique peut être appliqué sur un milieu (M) stocké ou retenu dans la zone de réaction ;
dans laquelle une surface d'électrode d'au moins une desdites électrodes opposées présente des parties creuses (3, 31, 32) ; et
une alimentation en courant (G) configurée pour appliquer un champ électrique sur un milieu dans la zone de réaction de sorte que l'acide nucléique pour la détection ou l'acide nucléique cible est déplacé à l'intérieur des parties creuses de manière à augmenter la concentration de l'acide nucléique.

2. Unité de détection d'hybridation selon la revendication 1,
dans laquelle lesdites parties creuses sont des parties destinées à fixer ledit acide nucléique pour la détection.

3. Unité de détection d'hybridation selon la revendication 1,
dans laquelle lesdites électrodes opposées sont revêtues d'une couche isolante (13, 15).

4. Unité de détection d'hybridation selon la revendication 3,
dans laquelle lesdites parties creuses sont formées dans ladite couche isolante.

5. Unité de détection d'hybridation selon la revendication 1,
dans laquelle lesdites parties creuses sont agencées régulièrement.

6. Unité de détection d'hybridation selon la revendication 1,
dans laquelle lesdites électrodes opposées sont disposées sur un côté supérieur et un côté inférieur avec ladite zone de réaction interposée entre lesdites électrodes opposées.

7. Unité de détection d'hybridation selon la revendication 6,
dans laquelle lesdites parties creuses sont formées sur au moins une desdites électrodes opposées.

8. Unité de détection d'hybridation selon la revendication 6,
dans laquelle au moins ladite électrode sur le côté inférieur est une électrode qui transmet une lumière d'excitation (P) d'une longueur d'onde prédéterminée.

9. Unité de détection d'hybridation selon la revendication 1,
dans laquelle lesdites électrodes opposées sont disposées sur un premier côté et un second côté de la zone de réaction de telle sorte que ladite zone de réaction est interposée entre lesdites électrodes opposées.

10. Unité de détection d'hybridation selon la revendication 1,
dans laquelle lesdites électrodes opposées sont formées de manière à être capables d'appliquer un champ de courant électrique alternatif haute fréquence sur ledit milieu.

11. Puce ADN (10) comprenant au moins l'unité de détection d'hybridation de la revendication 1.

12. Procédé de détection d'une hybridation entre l'acide nucléique pour la détection (D) et l'acide nucléique cible (T) dans un milieu (M), comprenant :
la fourniture d'une unité de détection d'hybridation (1a, 1b, 1c) comprenant :
une zone de réaction (2) destinée à fournir un champ pour l'hybridation entre l'acide nucléique pour la détection et l'acide nucléique cible ; et
des électrodes opposées (E1 ; E2, E3 ; E4) disposées de sorte qu'un champ électrique peut être appliqué sur un milieu stocké ou retenu dans la zone de réaction ;
dans laquelle une surface d'électrode d'au moins une desdites électrodes opposées présente des parties creuses (3, 31, 32) ;
l'introduction du milieu à l'intérieur de la zone de réaction ; et
l'application d'un champ électrique sur le milieu de sorte que l'acide nucléique pour la détection ou l'acide nucléique cible est déplacé à l'intérieur des parties creuses de manière à augmenter la concentration de l'acide nucléique.
